# EUROPEAN PATENT APPLICATION

(11) **EP 3 449 805 A1**
(43) Date of publication of application: **06.03.2019**
(21) Application number: 17788865.8
(22) Date of filing: 25.04.2017
(51) Int. Cl.: A61B 1/31

(54) **RESECTOSCOPE**

(30) Priority: 29.04.2016 ES 201630551
(71) Applicant: Fundación para la Investigación Biomédica del Hospital Universitario de la Paz (FIBHULP), 28046 Madrid (ES); Universidad Carlos III de Madrid, 28918 Leganés - Madrid (ES)
(72) Inventor: ÁLVAREZ GALLEGO, Mario, 28046 Madrid (ES); CASTEJÓN SISAMÓN, Cristina, 28918 Leganés (Madrid) (ES); GARCÍA PRADA, Juan Carlos, 28918 Leganés (Madrid) (ES); MENESES ALONSO, Jesús, 28918 Leganés (Madrid) (ES)
(74) Representative: Pons
(86) International application number: PCT/ES2017/070249
(87) International publication number: WO 2017/186994

(57) **Abstract**

Rectoscope designed to be inserted in the anorectal cavity of an individual with diagnostic and/or surgical purposes which incorporates a radial light source (13), gradually displaceable, which creates a radial beam of light with the object of transilluminating both the tubular body (1) of the rectoscope and the portion of the rectum wherein it is inserted to allow, in first place, a precise observation of the inside of said cavity and, in second place, in if any foreign body such a tumour is detected, to be surgically removed, it allows a precise determination of the distal resection margin to minimize the rectal portion removed.

## Description

### OBJECT OF THE INVENTION

The present invention is included in the technical field of instruments for performing a medical examination of the cavities or ducts of the body, specifically the rectum, by visual inspection or photography, and it relates in particular to a rectoscope which incorporates an adjustable lighting system to transilluminate the rectum, making it possible to optimize the observation of the rectal cavity for the detection of foreign bodies and, in the event that surgical intervention is required, the determination of the distal resection margin.

### BACKGROUND OF THE INVENTION

A rectoscope is a medical device of cylindrical and hollow geometry, designed to be inserted through the anus to view the inside of the rectum, with both diagnostic and surgical purposes. Since the rectum is a virtual cavity, i.e. in normal circumstances it is empty and collapsed, rectoscopes have means to insufflate air inside the rectum so that they distend its walls giving rise to an actual cavity.

Rectoscopes are known in the current state of the art equipped with illumination means, generally situated at one of the ends of the device, which create a beam of light in parallel to the major axis of the rectoscope cylinder, so that they illuminate the front of said rectoscope to look through it. Said illumination is usually considered sufficient to locate foreign bodies, such as, for example, tumours, in the patient's rectum.

Furthermore, rectal cancer surgery basically consists of removing the segment of the rectum that contains the tumour, and in joining or anastomosing the resulting free ends of the colon and rectum to maintain continuity in the passage of faeces.

When performing rectal resection, it is necessary to leave a safety margin between the tumour and the healthy rectal tissue, which is typically known as distal resection margin. Generally, it has been considered that 5 cm was a suitable distance, although the length of the martin has shortened over time and numerous recent studies demonstrate that a 1 cm margin is oncologically appropriate.

This reduction in distal resection margin is due, on the one hand to the fact that the defecatory function that the patient is going to experience after surgery is better the shorter the length of rectum removed and, on the other hand, that there is less risk of dehiscence. Dehiscence is a complication wherein the joining or anastomosis between the two ends of the colon and the rectum is separated and not suitably heal, with the consequence that the faecal content inside the rectum passes to the abdominal cavity and may cause a serious infection known as faecal peritonitis. This dehiscence is due to the fact that the rectum, the closer the margin is to the anus, the worse it is vascularized and, therefore, the healing processes, which are those that make the anastomosis link together suitably, are slower and more complex.

Said current trend to perform the rectal resection as close as possible to the tumour has the disadvantage that if it is done too close to the tumour (distance less than 1 cm), it does not guarantee oncological safety, since at times the edges of tumours are not clear and there may be intramural dissemination or deposits external to the tumour, and, therefore, on performing the rectal resection there would not be a total elimination of the tumoral tissue inside the rectum. Furthermore, if the rectal resection is performed directly on the tumour, it considerably increases the risk of tumoral tissue dissemination and local recurrence or repetition of the tumour.

### DESCRIPTION OF THE INVENTION

The object of the invention consists of a rectoscope to inspect the inside of the anorectal cavity of an individual, especially devised for exploratory and surgical procedures related to the presence of rectal tumours, which incorporates radial illumination means, which may be additional to those of the rectoscope if it has longitudinal illumination means, which enables optimising the observation of the rectal cavity for the detection of foreign bodies and, if surgical interventions are required, the precise determination of a distal resection margin.

The radial illumination means, preferably of moving type, make use of the translucent nature of the tissues forming the rectum, so that it is necessary that the rectoscope walls are made in a material that is also translucent, since in this way when said radial means emit a beam of light from the inside of the rectoscope, said beam passes both through the rectoscope walls and the tissues of the rectum wherein it is inserted, allowing observation of said beam from the inside of the abdominal cavity.

For this reason, the radial illumination means preferably comprise a rod on one of the ends whereof is disposed a ring-shaped structure in which outer end is disposed a plurality of light sources, preferably of LED type, to emit the beam of light. A housing for the power source is situated at the other end of the rod, in addition to the point from which the displacement of the rod through the inside of the rectoscope is controlled.

It contemplates the option of additionally incorporating front illumination means in the same angular structure wherein the radial illumination means are disposed. Said front illumination means, which like the radial ones as moving in character, may either complement the own longitudinal fixed illumination means of the rectoscope or replace them, in the case of those simple rectoscopes that do not have said illumination means.

The use of this device starts with the insertion by the surgeon's assistant of the rectoscope through the individual's anus, making it advance until the lower edge of the rectal tumour using the front illumination and insufflation. At that time, keeping the rectoscope tube in fixed position, the radial illumination means are activated and displaced along the major axis of the rectoscope. The length displaced shall be that estimated by the surgeon as distal resection margin.

With the radial light source transilluminating the rectum, the surgeon can view from inside the abdominal cavity, the point where he or she should perform the resection of the rectum at a distance from the tumour estimated by him or her as distal resection margin.

Finally, the surgical part is removed from the patient, with the tumour and the distal resection margin removed, with the rectal stump being prepared to proceed with the anastomosis.

The rectoscope thus described allows performing a precise observation of the inside of the individual's anorectal cavity and, if necessary to perform a surgical intervention to remove any foreign bodies detected, such as, for example, a tumour, to determine with a high degree of precision the distal resection margin, avoiding in this way the aforementioned drawbacks.

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:
Figure 1.- Shows a rear perspective view of the rectoscope, wherein its main constituent elements can be observed.
Figure 2.- Shows a side view of a cross-section made in the rectoscope.
Figure 3.- Shows a view according to the transversal axis of the rear part of the rectoscope, similar to that the health professional in charge of the exploration and/or surgical intervention observes.
Figure 4.- Shows a perspective view of a detail of the access window to the inside of the rectoscope, with the hatch open.
Figure 5.- Shows a perspective view of the second light source of the rectoscope.
Figure 6.- Shows a side view of a cross-section made in the second light source.
Figure 7.- Shows a perspective view of a detail of the second light source.

### PREFERRED EMBODIMENT OF THE INVENTION

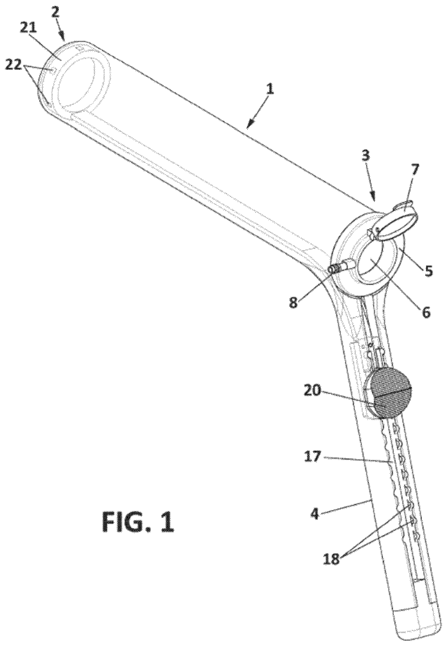

A detailed explanation of an example of preferred embodiment of the object of the present invention is provided below, with the aid of the aforementioned figures.
The rectoscope described is formed by a hollow tubular body (1), of essentially cylindrical geometry and made in a translucent material, comprising an open first end (2), designed to be inserted in the rectal region of an individual, and a second end (3) wherefrom a hand grip (4) emerges to allow the introduction of the rectoscope in the individual and its later handling, as shown in figure 1.

The second end (3) of the tubular body (1) additionally comprises an access window (5), of essentially ring-shaped geometry and diametric dimensions similar to those of said second end (3), to facilitate the connection between both elements. The ring-shaped geometry of the access window (5) defines in its central part an opening (6) wherethrough the inside of the tubular body (1) is accessed. Said opening (6) is additionally equipped with a moving hatch (7) to regulate said access to the inside of the tubular body (1).

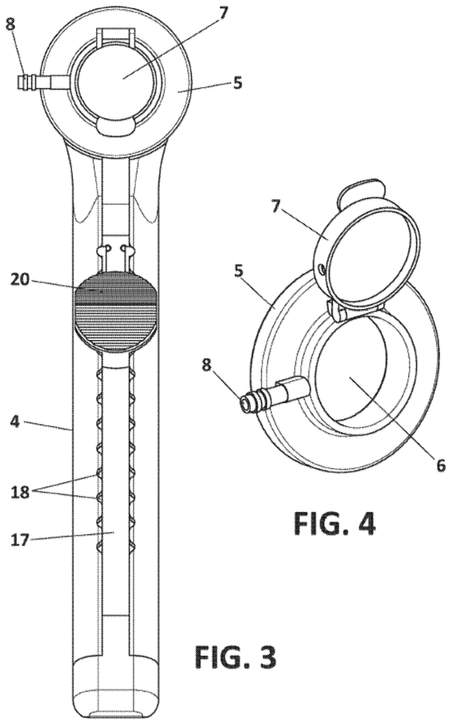

In the preferred embodiment described here, the hatch (7) is hinged by means of a hinge, as observed in attached figures 1 to 3. It additionally contemplates that said hatch (7) is made in a material that acts as magnifying lens, to facilitate observation through it.

The access window (5) further comprises a nozzle (8) designed to be connected to an insufflator, not represented in the attached figures, to in this way allow the passage of an airflow from said insufflator through the inside of the tubular body (1) for its exit through the first end (2), producing the distension of the individual's rectum walls.

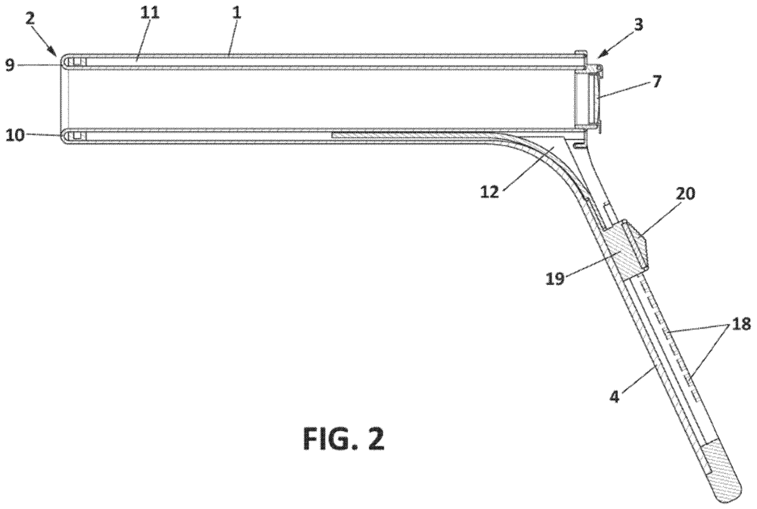

As observed in figure 2, the tubular body (1) comprises the joining of a first outer tube (9) and a second inner tube (10), concentric to the first tube (9). The difference between the diametric dimensions of the first tube (9) and the second tube (10) creates a first hollow channel (11) in the wall of the tubular body (1).

Inside the hollow tubular body (1), in an area close to its second end (3) as shown in figure 2, a fixed light source (12) is disposed, designed to create a first longitudinal beam of light in parallel to the major axis of the tubular body (1) of the rectoscope, so that said first beam of light illuminates both the first end (2) and the rectal region of the individual with whom it is in contact. The fixed light source (12) comprises a plurality of high power LED diodes.

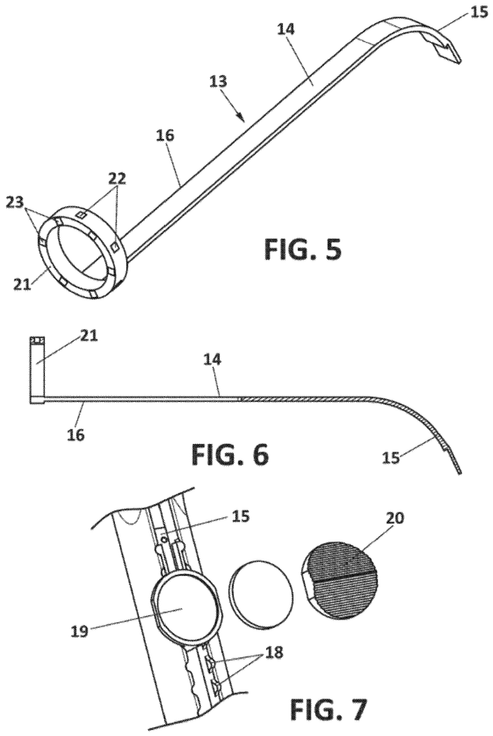

The rectoscope incorporates a moving radial light source (13), designed to create a second beam of radial-type light to transilluminate both the tubular body (1) and the portion of the rectum wherein said tubular body (1) is inserted, since both are bodies of translucent nature. To do this, as illustrated in figures 5 and 6, the radial light source (13) comprises a rod (14) of flexible nature which displaces inside the first channel (11) of the tubular body (1), said rod (14) being equipped with a third end (15) and a fourth end (16) The third end (15) of the rod (14) is designed to displace inside a second longitudinal channel (17) defined in the hand grip (4). As observed in the attached figures, said second channel (17) comprises a plurality of protuberances (18), similar to saw-teeth, defined in its sides to discretize and graduate the displacement of the rod (14) through the inside of the second channel (17).

Said third end (15) additionally comprises a housing (19) to house a power source of the radial light source (13). The housing (19) comprises a hinged lid (20) which in first place allows access to its inside to be able to perform maintenance tasks, such as, for example, the replacement of its power source, and in second place it acts as support point for the manual displacement of the third end (15), and in consequence of the rod (14), throughout the second channel (17) of the hand grip (4). This displacement is graduated by the lateral contact of the housing (19) with the protuberances (18) of the second channel (17).

The fourth end (16) of the rod (14) is solidly joined to a ring-shaped support (21) of dimensions slightly less than those of the first channel (11) of the tubular body (1), wherethrough it is designed to displace. A plurality of light sources (22) uniformly distributed on the outer face of the ring-shaped support (21) emit a second radial beam of light which passes through the translucent walls of the tubular body (1) and the portion of the individual's rectum wherein said tubular body (1) is inserted.

In the preferred embodiment described here, the ring-shaped support (21) incorporates in its front part a moving front light source (23), which emits a third longitudinal beam of light which complements the first longitudinal beam of light emitted by the fixed light source (12) of the rectoscope.

The rectoscope described here is inserted in the individual's rectum through the anus.

The manual insufflator introduces an airflow through the nozzle (8) inside the tubular body (1), which exits through the first end (2) and distends the walls of the individual's rectum.

When a tumour is located inside the rectum, thanks to the first beam of light created by the fixed light source (12), the tubular body (1) of the rectoscope is disposed so that its first end (2) is situated in a position adjacent to said tumour.

In that time, the third end (15) of the rod (14) manually displaces throughout the second channel (17) of the hand grip (4). The displacement is performed until situating the ring-shaped support (21) in a position at a distance from (2) that the surgeon has determined, this being the distal resection margin.

With the radial light source (13) emitting the second beam of light through the tubular body and the rectum, a surgeon can view the point where the cut starts, observing an optimal distal resection margin, overcoming in this way the aforementioned problems associated to inadequate length margins. If required, the third beam of light emitted by the front light source (23) can contribute to making said display even more precise.

The opening of the hatch (7) additionally allows introduction of the surgical material designed to physically remove the tumour located in this way and the performance of cleaning operations in the hatch (7).

## Claims

1. Rectoscope designed to be inserted in the anorectal cavity of an individual with diagnostic and/or surgical purposes, comprising:
- a tubular body (1) of essentially cylindrical and hollow geometry and with translucent walls, comprising in turn a first end (2) intended to be inserted inside the anorectal cavity of an individual and a second end (3) intended to be disposed in the outer part of said cavity,
- a hand grip (4) which is projected from the second end (3) of the tubular body (1) for the introduction and handling of the rectoscope, and
- an access window (5) of essentially ring-shaped geometry designed to be connected to the second end (3) of the tubular body (1) to regulate access to the inside of said tubular body (1),
**characterized in that** it additionally incorporates a moving radial light source (13), displaceable inside the tubular body (1), designed to create a beam of radial-type light to transilluminates both the tubular body (1) and the portion of the rectum wherein said tubular body (1) is inserted.

2. Rectoscope according to claim 1, wherein the radial light source (13) comprises:
- a rod (14) which displaces inside the tubular body (1), comprising in turn a third end (15) and a fourth end (16),
- a housing (19) defined in the third end (15) to house a power source of the radial light source (13),
- a hinged lid (20) to cover the housing (19),
- a ring-shaped support (21), solidly joined to the fourth end (16) of the rod (14), of dimensions slightly less than those of the interior of the tubular body (1), and
- a plurality of light sources (22) uniformly distributed on the outer face of the ring-shaped support (21), designed to emit the radial beam of light.

3. Rectoscope according to claim 1, further comprising a moving front light source (23), designed to emit a longitudinal beam of light parallel to the axis of the tubular body (1).

4. Rectoscope according to claims 2 and 3, wherein the front light source (23) is disposed in the front face of the ring-shaped support (21).

5. Rectoscope according to claim 1, wherein the access window (5) additionally comprises:
- an opening (6) defined in the centre of the access window (5),
- a retractive hatch (7) to cover the opening (6), and
- a nozzle (8) designed to connected to an insufflator.

6. Rectoscope according to claim 1, wherein the tubular body (1) comprises the joining of a first outer tube (9) and a second inner tube (10), concentric to the first tube (9) and of smaller diametric dimensions.

7. Rectoscope according to claims 2 and 6, wherein the difference between the diametric dimensions of the first tube (9) and the second tube (10) creates a first hollow channel (11) in the wall of the tubular body (1) wherethrough the rod (14) displaces.

8. Rectoscope according to claim 1, wherein the hand grip (4) additionally incorporates a second channel (17) defined longitudinally on its surface, and a plurality of protuberances (18) defined in the sides of the second channel (17).

9. Rectoscope according to claims 2 and 8, wherein the third end (15) of the rod (14) is of flexible nature to adapt to the curved geometry of the second channel (17) of the hand grip (4).

10. Rectoscope according to claims 2 and 8, wherein the protuberances (18) are distanced from one another in a graduated and uniform manner to discretize the displacement of the third end (15) of the rod (11) throughout the second channel (17).

11. Rectoscope according to claim 2, wherein the light sources (22) are LED diodes.

12. Rectoscope according to claim 3, wherein the front light source (23) are LED diodes.
